# EUROPEAN PATENT APPLICATION

(11) **EP 4 417 229 A1**
(43) Date of publication of application: **21.08.2024**
(21) Application number: 22879847.6
(22) Date of filing: 25.05.2022
(51) Int. Cl.: A61M 5/142

(54) **MEDICAL SURGICAL PUMP, AND METHOD FOR CONTROLLING TUBE APPLIED TO MEDICAL SURGICAL PUMP**

(30) Priority: 15.10.2021 CN 202111204088
(71) Applicant: Shenzhen Mindray Bio-Medical Electronics Co., Ltd., Shenzhen, Guangdong 518057 (CN)
(72) Inventor: PENG, Mingxu, Shenzhen, Guangdong 518057 (CN); ZUO, Pengfei, Shenzhen, Guangdong 518057 (CN); ZHANG, Peng, Shenzhen, Guangdong 518057 (CN)
(74) Representative: KIPA AB
(86) International application number: PCT/CN2022/094991
(87) International publication number: WO 2023/060898

(57) **Abstract**

A medical surgical pump, and a method for controlling a tube (210) applied to a medical surgical pump. The medical surgical pump comprises a first pressing member (310), a second pressing member (320), a first driving assembly (390), and a trigger unit (370). The first pressing member (310) and the second pressing member (320) are used to press the tube (210), so as to achieve the aim of tube locking. The first driving assembly (390) is connected to the first pressing member (310) and/or the second pressing member (320). Before tube mounting, there is a relatively large gap (330) between the first pressing member (310) and the second pressing member (320), thereby reducing the difficulty of mounting a tube by a user. After tube mounting, the trigger unit (370) controls at least one of the first pressing member (310) and the second pressing member (320) to move, such that the first pressing member (310) and the second pressing member (320) clamp the tube (210), thereby achieving tube locking.

## Description

### TECHNICAL FIELD

The disclosure relates to a field of medical devices, in particular to an structure for assembling and disassembling a pipeline of a medical surgical pump.

### BACKGROUND

Medical surgical pump, as a device which is capable of controlling a flowing of a fluid (including a gas and a liquid), is widely used in medical environments, such as a uterine distension pump, a flushing pump, a suction pump, or a flushing and suction integrated pump, which are used in an endoscope surgery.

During an endoscope surgery, a flushing pump, a suction pump (or a flushing and suction integrated pump) is needed to flush a wound and suck out waste fluid, so as to maintain a clear field of vision. During preoperative preparation, a perfusion tube and a suction tube need to be connected with and used in conjunction with the flushing and suction integrated pump.

In present art, a rolling-squeezing method to stop a liquid and lock a pipeline. Specifically, the liquid is stopped by squeezing the pipeline through a liquid-stop wall and a roller. The advantage of the rolling-squeezing method is that the liquid discharge is more stable, but its traditional assembly scheme for the pipeline is generally multi-step assembly, which requires steps, such as plugging the pipeline and fixing it. Therefore, it is time-consuming and labour-intensive, and reduces an efficiency of surgery.

### SUMMARY

### Technical problem

This disclosure provides a new medical surgical pump, and a control method which is applied to a pipeline of a medical surgical pump, so as to demonstrate a new way for assembling and disassembling a pipeline.

### Technical Solution

Based on above purpose, an embodiment of this disclosure provides a medical surgical pump, including a housing, a control unit, a first driving component, a first squeezing element, and a second squeezing element;
the housing includes a panel, wherein the first squeezing element and/or the second squeezing element are/is movably arranged on the panel; the control unit is connected with the first driving component, and the first driving component is further connected with the first squeezing element and/or the second squeezing element; wherein a gap, which is configured to accommodate a pipeline, exists between the first squeezing element and the second squeezing element;
the control unit is configured to control the first driving component to drive at least one of the first squeezing element and the second squeezing element to move toward the other, so as to decrease the gap for fixing the pipeline, when receiving a first trigger signal; and/or the control unit is configured to control the first driving component to drive at least one of the first squeezing element and the second squeezing element to move away from the other, so as to increase the gap for releasing the pipeline, when receiving a second trigger signal.

In one embodiment, the medical surgical pump further includes at least one first in-position detection unit, and the first trigger signal includes a trigger signal for assembling a pipeline;
the first in-position detection unit is configured to transmit the trigger signal for assembling a pipeline to the control unit, when detecting that the pipeline is being assembled;
the control unit is further configured to control the first driving component to drive at least one of the first squeezing element and the second squeezing element to move toward the other, so as to decrease the gap, when receiving the trigger signal for assembling a pipeline.

In one embodiment, the medical surgical pump further includes at least one first in-position detection unit, and the second trigger signal includes a trigger signal for disassembling a pipeline;
the first in-position detection unit is configured to transmit the trigger signal for disassembling a pipeline to the control unit, when detecting that the pipeline is being disassembled;
the control unit is further configured to control the first driving component to drive at least one of the first squeezing element and the second squeezing element to move away from the other, so as to increase the gap, when receiving the trigger signal for disassembling a pipeline.

In one embodiment, the first in-position detection unit is arranged inside a pipeline accommodating region of the medical surgical pump.

In one embodiment, the medical surgical pump further includes a unit for inputting a user instruction, wherein the first trigger signal includes a trigger signal for assembling a pipeline;
the unit for inputting a user instruction is configured to transmit the trigger signal for assembling a pipeline to the control unit, when receiving, from a user, an instruction for assembling a pipeline;
the control unit is further configured to control the first driving component to drive at least one of the first squeezing element and the second squeezing element to move toward the other, so as to decrease the gap, when receiving the trigger signal for assembling a pipeline.

In one embodiment, the medical surgical pump further includes a unit for inputting a user instruction, wherein the second trigger signal includes a trigger signal for disassembling a pipeline;
the unit for inputting a user instruction is configured to transmit the trigger signal for disassembling a pipeline to the control unit, when receiving, from a user, an instruction for disassembling a pipeline;
the control unit is further configured to control the first driving component to drive at least one of the first squeezing element and the second squeezing element to move away from the other, so as to increase the gap, when receiving the trigger signal for disassembling a pipeline.

In one embodiment, the unit for inputting a user instruction includes at least one of a physical trigger element, a touch-control element, a sound acquisition unit, and a gesture acquisition unit.

In one embodiment, the medical surgical pump further includes a trigger unit, wherein the first trigger signal includes a trigger signal for assembling a pipeline, and the second trigger signal includes a trigger signal for disassembling a pipeline;
the trigger unit is configured to transmit the trigger signal for assembling a pipeline to the control unit, when detecting that the pipeline is being assembled or receiving from a user an instruction for assembling a pipeline; the control unit is further configured to control the first driving component to drive at least one of the first squeezing element and the second squeezing element to move toward the other, so as to decrease the gap, when receiving the trigger signal for assembling a pipeline; and/or
the trigger unit is configured to transmit the trigger signal for disassembling a pipeline to the control unit, when detecting that the pipeline is being disassembled or receiving from a user an instruction for disassembling a pipeline; the control unit is further configured to control the first driving component to drive at least one of the first squeezing element and the second squeezing element to move away from the other, so as to increase the gap, when receiving the trigger signal for disassembling a pipeline.

In one embodiment, the medical surgical pump further includes a position detection unit, which is arranged on moving track(s) of the first squeezing element and/or the second squeezing element, wherein the position detection unit is connected with the control unit;
the position detection unit is configured to transmit an in-position signal to the control unit, when detecting that the first squeezing element and/or the second squeezing element move/moves into position;
the control unit is further configured to control the first driving component to stop driving the first squeezing element and/or the second squeezing element to move, when receiving the in-position signal.

In one embodiment, at least a section of a relative movement between the first squeezing element and the second squeezing element is a translational movement.

In one embodiment, the first squeezing element includes an arc-shaped surface, and the second squeezing element includes a roller component;
the roller component includes a rotating wheel and a plurality of squeezing wheels, wherein the squeezing wheels are arranged around the rotating wheel.

In one embodiment, the medical surgical pump further includes a second driving component, which is connected with the roller component;
the second driving component is configured to drive the rotating wheel to rotate, so as to enable the squeezing wheels, when abutting against the pipeline, to move along the arc-shaped surface, thereby pushing a fluid inside the pipeline to flow.

In one embodiment, the second squeezing element further includes a holder, wherein the roller component is assembled at the holder, the first driving component includes a motor and a transmission cam, an output shaft of the motor is connected with the transmission cam, the transmission cam is further connected with the holder, wherein a rotation of the transmission cam is capable of driving the holder to move toward and/or away from the first squeezing element.

In one embodiment, the medical surgical pump further includes a locking element and a third driving component, wherein the housing is provided with a base, and the base is provided with a placement region, which is configured to place a pipeline component; wherein the pipeline component includes the pipeline and a fixing block, wherein the pipeline is assembled at the fixing block, and the locking element is configured to lock and unlock the fixing block of the pipeline component; wherein the third driving component is connected with the locking element, so as to drive the locking element to move between a locking position and an unlocking position;
when the locking element is at the locking position, the locking element and the placement region form a position-limiting space, which is capable of accommodating the pipeline component, and the locking element is capable of locking the fixing block inside the position-limiting space; when the locking element is at the unlocking position, the locking element unlocks the fixing block.

In one embodiment, when the locking element is switched to the locking position, the first trigger signal is transmitted to the control unit; and/or when the locking element is switched to the unlocking position, the second trigger signal is transmitted to the control unit.

In one embodiment, the medical surgical pump further includes a second in-position detection unit, which is connected with the control unit;
the second in-position detection unit is configured to trigger the control unit to control the third driving component, so as to drive the locking element to move to the locking position, when detecting that the pipeline component is arranged inside the placement region.

Based on above purpose, an embodiment of this disclosure provides a control method which is applied to a pipeline of a medical surgical pump, including:
controlling at least one of a first squeezing element and a second squeezing element to move toward the other, so as to clamp the pipeline, when a first trigger signal is received; and/or
controlling at least one of a first squeezing element and a second squeezing element to move away from the other, so as to release the pipeline, when a second trigger signal is received.

In one embodiment, the control method further includes:
determining that the first trigger signal is received, when it is detected that the pipeline is being assembled into a pipeline accommodating region of the medical surgical pump; and/or
determining that the second trigger signal is received, when it is detected that the pipeline is being disassembled out of a pipeline accommodating region of the medical surgical pump.

In one embodiment, the control method further includes:
determining that the first trigger signal is received, when an instruction for assembling a pipeline is received; and/or
determining that the second trigger signal is received, when an instruction for disassembling a pipeline is received.

Based on above purpose, an embodiment of this disclosure provides a computer-readable storage medium, including program(s), which is/are capable of being executed by a processor, so as to implement any of the control methods described above.

### Beneficial Effects

The medical surgical pump according to above embodiment(s) includes a first squeezing element, a second squeezing element, a first driving component and a trigger unit. The first squeezing element and the second squeezing element are configured to squeeze the pipeline for locking the pipeline. The first driving component is connected with the first squeezing element and/or the second squeezing element. Before the pipeline is assembled, a large gap exists between the first squeezing element and the second squeezing element, which decreases the difficulty of assembling the pipeline for the user. After the pipeline is being assembled, the trigger unit controls at least one of the first squeezing element and the second squeezing element to move, so as to enable the first squeezing element and the second squeezing element to clamp the pipeline for locking the pipeline.

### BRIEF DESCRIPTION OF THE DRAWINGS

FIG. 1 is a structural diagram for two states of unlocking and locking a pipeline, in one embodiment of this disclosure.
FIG. 2 is a diagram in which a pipeline is squeezed by a movement of a first squeezing element, in one embodiment of this disclosure.
FIG. 3 is a diagram in which a pipeline is squeezed by a movement of a second squeezing element, in one embodiment of this disclosure.
FIG. 4 is a top view (upper) and a rear view (lower) of a pipeline-unlocked state, in one embodiment of this disclosure.
FIG. 5 is a top view (top) and a rear view (bottom) of a pipeline-locked state, in an embodiment of this disclosure.
FIG. 6 is a linkage diagram with a locking element in both pipeline-unlocked state and pipeline-locked state, in one embodiment of this disclosure.
FIG. 7 is a structural diagram in which a fixing block is locked, in one embodiment of this disclosure.
FIG. 8 is a diagram in which a locking element is at an unlocking position according to an embodiment of this disclosure.
FIG. 9 is a diagram in which a locking element is at a locking position according to an embodiment of this disclosure.
FIG. 10 is a diagram showing connection(s) of some modules, according to one embodiment of this disclosure.

### DESCRIPTION OF THE PREFERRED EMBODIMENTS

This disclosure is further described in detail below through specific embodiments in combination with the accompanying drawings. In different embodiments, similar elements adopt associated similar reference numbers. In the following embodiments, many details are described in order to make this disclosure better understood. However, one skilled in the art can easily recognize that some of the features can be omitted in different cases, or can be replaced by other elements, materials and methods. In some cases, some operations related to this disclosure are not shown or described in the description, in order to avoid the core part of this disclosure being inundated by too many descriptions. For one skilled in the art, it is not necessary to describe these related operations in detail, as they can fully understand the relevant operations according to the description in this disclosure and the general technical knowledge in the art.

In addition, the features, operations, or characteristics described in the description may be combined in any appropriate manner to form various embodiments. At the same time, the steps or actions in the method description can also be exchanged or adjusted sequentially in a manner obvious to one skilled in the art. Therefore, the various sequences in the description and the drawings are only for the purpose of clearly describing a certain embodiment, and do not mean that they are necessary sequences. Unless it is otherwise specified that one of the sequences must be followed.

The terms "first", "second", etc. in the description are operable to distinguish different objects, rather than to describe a specific order. In addition, the terms "connection", and "linkage" mentioned in this disclosure include direct and indirect connection (linkage), unless otherwise specified.

Embodiments provide a pipeline fixing apparatus for a medical surgical pump, which is a pump used in a surgical medical environment, such as a uterine distension pump, a flushing pump, a suction pump, a flushing and suction integrated pump, etc., used in endoscope surgery. The pipeline fixing apparatus can be configured to lock the pipeline in a surgical pump, and can even be configured to stop a fluid inside the pipeline. Alternatively, in one embodiment, the pipeline fixing apparatus can also push the fluid inside the pipeline to flow.

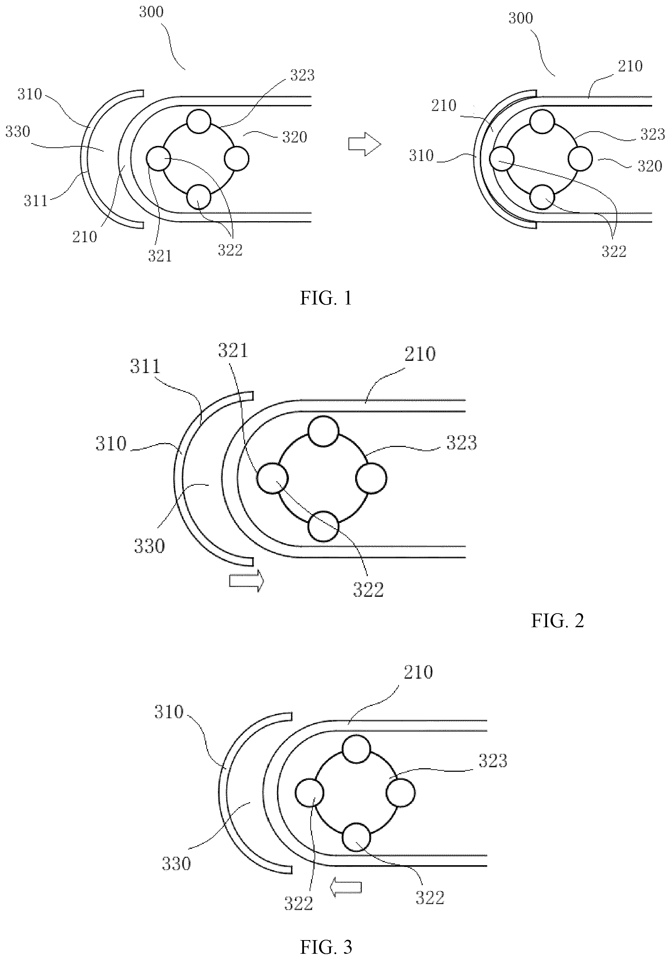

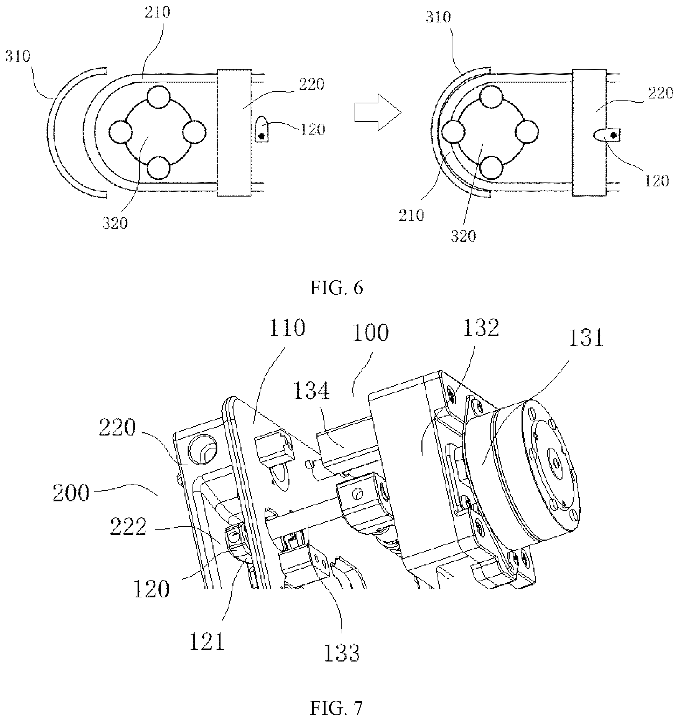

Please refer to FIGS. 1-6 and 10. In one embodiment, the pipeline fixing apparatus 300 includes a first squeezing element 310, a second squeezing element 320, a first driving component 390, a control unit 380, and a trigger unit 370. In addition, the medical surgical pump is provided with a housing (a base 110 in FIG. 7 is a part of the housing), wherein the housing has a cavity, the control unit 380 and the first driving component 390 are arranged inside the cavity. As shown in FIG. 7, the housing is provided with a panel, the first squeezing element 310 and the second squeezing element 320 are arranged on the panel for fixing the pipeline.

The first squeezing element 310 is provided with a first squeezing portion 311 for squeezing the pipeline 210, and the second squeezing element 320 is provided with a second squeezing portion 321 for squeezing the pipeline 210. A gap 330, which is wider than an outer diameter of the pipeline 210, exists between the second squeezing portion 321 and the first squeezing portion 311. As shown in FIG. 1, the gap 330 can be much larger than the outer diameter of the pipeline 210, so that the user can easily place the pipeline 210 between the second squeezing portion 321 and the first squeezing portion 311.

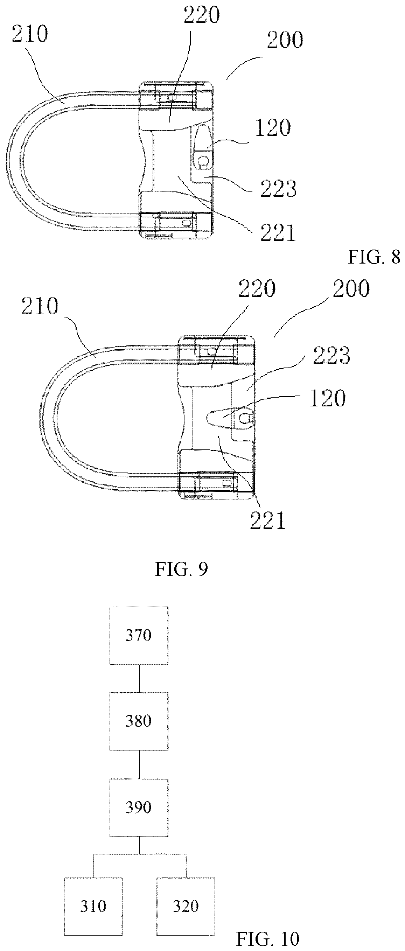

The first driving component 390 is connected with the first squeezing element 310 and/or the second squeezing element 320, and is configured to drive at least one of the first squeezing element 310 and the second squeezing element 320 to move toward a position of the pipeline 210, so as to squeeze the pipeline 210. In the embodiment shown in FIG. 10, the first driving component 390 can drive the first squeezing element 310 and the second squeezing element 320 to move. In fact, in addition to this, the first driving component 390 can either only drive the first squeezing element 310 to move (as shown in FIG. 2) or only drive the second squeezing element 320 to move (as shown in FIG. 3). The ultimate goal is to move the first squeezing element 310 and the second squeezing element 320 to be relatively close to each other, so as to decrease the gap 330 between the second squeezing portion 321 and the first squeezing portion 311, and finally make the gap 330 to be smaller than the outer diameter of the pipeline 210, as shown in a structure on the right side of FIG. 1, thereby squeezing the pipeline 210 for stopping the liquid and locking the pipeline.

The control unit 380 may select a commonly used controller, which is capable of receiving, processing, and transmitting information, such as a processor, a memory, etc. In addition, the control unit 380 may be other control apparatus without a processor, such as a single-chip microcomputer, an analogy circuit (switch control), and the likes. The control unit 380 may be a control unit 380 of the medical surgical pump itself, or may be another control unit 380 which is different from the control unit 380 of the medical surgical pump itself. The control unit 380 is connected with the first driving component 390, so as to control the first driving component 390 to work. The trigger unit 370 is connected with the control unit 380 and transmits a first trigger signal to the control unit 380. The first trigger signal includes a trigger signal for assembling a pipeline. When the control unit 380 receives the trigger signal for assembling a pipeline, it drives at least one of the first squeezing element 310 and the second squeezing element 320 to move, so as to enable the first squeezing element 310 and the second squeezing element 320 to clamp the pipeline 210 for stopping the liquid and locking the pipeline.

The first driving component 390 includes a driving element 131 and a transmission element 133. The driving element 131 is connected with the transmission element 133. The transmission element 133 directly or indirectly transmits a movement, which is outputted by the driving element 131, to the first squeezing element 310 and/or the second squeezing element 320, so as to drive the first squeezing element 310 and/or the second squeezing element 320 to move. The driving element 131 is not limited to a motor, a cylinder, a hydraulic cylinder, an electromagnet, etc. The transmission element 133 can adopt a common transmission structure, such as a shaft-transmission structure, a synchronous belt-transmission mechanism, a gear-transmission mechanism and other transmission mechanisms and combination(s) thereof.

Before the pipeline is assembled, a large gap 330 exists between the first squeezing element 310 and the second squeezing element 320, which decreases the difficulty of assembling the pipeline for the user and provides an avoidance space for the pipeline 210, enables the user to assemble the pipeline directly without having to plug the pipeline 210 into a small space. After the pipeline is being assembled, the trigger unit 370 transmits a first trigger signal to the control unit 380. When the control unit 380 receives the first trigger signal, or the trigger unit 370 directly transmits the first trigger signal, at least one of the first squeezing element 310 and the second squeezing element 320 is driven to move, so as to enable the first squeezing element 310 and the second squeezing element 320 to clamp the pipeline 210 for stopping the liquid and locking the pipeline, so that the surgical pump enters into a working state.

The pipeline fixing apparatus 300 is easy to operate for the user, when assembling the pipeline, steps of manually plugging the pipeline (i.e., plugging the pipeline 210 into a space smaller than the outer diameter of the pipeline 210) and fixing the pipeline 210, are omitted, thereby improving the convenience of use.

The trigger unit 370 may be triggered by automatic detection or by manually inputting an instruction through a user.

For example, in one embodiment, the trigger unit 370 includes at least one first in-position detection unit 351, which is configured to detect whether the pipeline 210 is assembled in position, and is further configured to transmit a trigger signal for assembling a pipeline when detecting that the pipeline 210 is assembled in position. When detecting that the pipeline 210 is not assembled in position, the first in-position detection unit 351 may not transmit a trigger signal, or may transmit a signal which indicates that the pipeline is not in position.

The first in-position detection unit 351 can detect the pipeline 210 in various ways and principles, such as but not limited to detection apparatuses, such as an optical coupler, a potentiometer, a magnetic angle sensor and the likes.

In one embodiment, the first in-position detection unit 351 may be arranged inside a pipeline accommodating region of the medical surgical pump. That is, the pipeline accommodating region for placing the pipeline 210 is reserved at the housing of the medical surgical pump, and the first in-position detection unit 351 can be arranged inside the pipeline accommodating region, and as long as when the pipeline 210 is placed inside the pipeline accommodating region, it can be detected by the first in-position detection unit 351.

In order to more accurately know whether the pipeline is actually assembled, in one embodiment, the first in-position detection unit 351 is arranged inside the gap 30 between the second squeezing portion 321 and the first squeezing portion 311. That is, only when the first in-position detection unit 351 detects that the pipeline 210 is placed inside the gap 330 between the second squeezing portion 321 and the first squeezing portion 311, it is considered that an operation of assembling a pipeline is required. This method can prevent the first in-position detection unit 351 at other positions from being accidentally touched and mistakenly determined as assembling a pipeline is required.

In one embodiment, the trigger unit 370 may also be a unit for inputting a user instruction, which unit is configured to receive instruction(s) inputted by a user, and the unit for inputting a user instruction is connected with the control unit 380. The instruction(s) inputted by the user may include an instruction for assembling a pipeline. In one embodiment, when the unit for inputting a user instruction receives the instruction for assembling a pipeline, which instruction is inputted by the user, a trigger signal for assembling a pipeline is transmitted to the control unit 380. The control unit 380 receives the trigger signal for assembling a pipeline and controls the driving component to lock the pipeline.

The unit for inputting a user instruction can be implemented by various structures that can realize an input of user instruction. For example, in one embodiment, the unit for inputting a user instruction includes one or more of a physical trigger element (such as a physical key, a lever, a block, a knob, etc.), a touch-control element (such as a touch-control key, a touch-control screen, etc.), a sound acquisition unit (such as a voice control unit, which controls a working state of the driving component by voice, etc.), and a gesture acquisition unit (such as controlling the working state of the driving component by gesture recognition, etc.).

In the above embodiments, the trigger unit 370 can transmit a trigger signal for assembling a pipeline automatically or in response to a user instruction, so as to enable the control unit 380 to control the driving component for locking the pipeline. When the pipeline needs to be dissembled, the control unit 380 can control the driving component to drive at least one of the first squeezing element 310 and the second squeezing element 320 to move, so as to enable the first squeezing element 310 and the second squeezing element 320 to release the pipeline 210. Specifically, the control unit 380 is further configured to control the first driving component 390 to drive at least one of the first squeezing element 310 and the second squeezing element 320 to move away from the other, so as to increase the gap 330, after receiving the second trigger signal which is transmitted by the trigger unit 370.

Specifically, in one embodiment, the second trigger signal includes a trigger signal for disassembling a pipeline. When the trigger unit 370 detects that the pipeline 210 is assembled in position or when the trigger unit 370 receives an instruction for assembling a pipeline inputted by the user, the trigger unit 370 transmits a trigger signal for assembling a pipeline to the control unit 380. The control unit 380 receives the trigger signal for assembling a pipeline and controls the driving component to lock the pipeline.

When the trigger unit 370 detects that the pipeline 210 is at least partially dissembled or receives an instruction for disassembling a pipeline inputted by the user, it transmits a trigger signal for disassembling a pipeline to the control unit 380. The control unit 380 receives the trigger signal for disassembling a pipeline and controls the first driving component 390 to release the pipeline 210.

For example, in one embodiment, the first in-position detection unit 351 is configured to detect whether at least a portion of the pipeline 210 is being dissembled, and to transmit a trigger signal for disassembling a pipeline to the control unit 380, when determining that at least a portion of the pipeline 210 is being dissembled. When the control unit 380 receives the trigger signal for disassembling a pipeline, it drives at least one of the first squeezing element 310 and the second squeezing element 320 to move, so as to enable the first squeezing element 310 and the second squeezing element 320 to release the pipeline 210.

That the first in-position detection unit 351 is configured to detect whether at least a portion of the pipeline 210 is being dissembled, means that as long as a portion of the pipeline 210 is detected to be out of its placement position, the pipeline 210 is determined to be expected to be disassembled.

Of course, in order to more accurately determine whether it is really expected to disassemble the pipeline, in one embodiment, only when the pipeline 210 moves more than a preset distance from its placement position, the pipeline 210 is determined to be expected to be disassembled. The preset distance can be specifically set according to actual conditions. In this way, it is possible to avoid the situation where the pipeline 210 is mistakenly considered to need to be disassembled due to disturbance.

In order to better detect whether the pipeline 210 needs to be disassembled, in one embodiment, at least one trigger unit 370 is arranged inside a pipeline accommodating region for accommodating a portion of the pipeline 210, which portion is located outside the second squeezing portion 321 and the first squeezing portion 311. That is, this embodiment determines whether the pipeline 210 is expected to be disassembled by detecting whether the portion of the pipeline 210, which portion is not clamped by the second pressing portion 321 and the first pressing portion 311, is disassembled.

In another embodiment, an action for disassembling a pipeline may also be triggered by a user inputted instruction. For example, when the unit for inputting a user instruction receives from a user an instruction for disassembling a pipeline, the unit for inputting a user instruction transmits the trigger signal for assembling a pipeline to the control unit 380. The control unit 380 receives the trigger signal for disassembling a pipeline, and controls the first driving component 380 to release the pipeline 210.

Of course, in other embodiments, when disassembling the pipeline, the first driving component 390 and the first squeezing element 310 and/or the second squeezing element 320 can be temporarily out of a linkage state, and the user can manually push the first squeezing element 310 and/or the second squeezing element 320 away to disassemble the pipeline 210. For example, when the driving element 131 of the first driving component 390 is a motor, the cam is driven by the motor, and at least one of the first squeezing element 310 and the second squeezing element 320 can be pushed out by utilizing a protrusion with a longer radius on the cam. When the protrusion with a longer radius on the cam turns to another position and an arc portion with a shorter radius faces the first squeezing element 310 and/or the second squeezing element 320, at this time, the first squeezing element 310 and/or the second squeezing element 320 are released, and the user can manually push the first squeezing element 310 and/or the second squeezing element 320 to reset, thereby releasing the pipeline 210.

Alternatively, the resetting when the pipeline is disassembled can be accomplished by acting on the first squeezing element 310 and/or the second squeezing element 320 through an elastic element. For example, when the first squeezing element 310 and the second squeezing element 320 release the pipeline 210, the elastic element is in a squeezed or stretched state. When the pipeline is disassembled, the driving component is released from the first squeezing element 310 and/or the second squeezing element 320, and then the elastic element drives the first squeezing element 310 and/or the second squeezing element 320 to reset under its own elastic force.

Furthermore, in order to better control a movement stroke of the first squeezing element 310 and/or the second squeezing element 320, in one embodiment, a position detection unit 352 is also included. The position detection unit 352 is arranged on moving track(s) of the first squeezing element 310 and/or the second squeezing element 320 to detect whether the first squeezing element 310 and/or the second squeezing element 320 move(s) into position; wherein the position detection unit 352 is connected with the control unit 380.

The position detection unit 352 can detect moving position(s) of the first squeezing element 310 and/or the second squeezing element 320 through various methods and principles, such as but not limited to an optical coupler, a potentiometer, a magnetic angle sensor and the likes.

In one embodiment, when the position detection unit 352 detects that the first squeezing element 310 and/or the second squeezing element 320 move(s) into position, an in-position signal is transmitted to the control unit 380, and the control unit 380 controls the first driving component 390 to stop movement(s) of the first squeezing element 310 and/or the second squeezing element 320, or to keep the first squeezing element 310 and/or the second squeezing element 320 at a current position, or the control unit 380 may also perform other operations.

Further, referring to FIGS. 1-3, in one embodiment, the first squeezing element 310 has a first squeezing portion 311, and the first squeezing element 310 has an arc-shaped surface. The second squeezing element 320 includes a roller component, which is provided with at least one arc-shaped or circular protrusion 322 protruding outward, wherein an outer wall of the protrusion 322 has an arc-shaped second squeezing portion 321.

In some embodiments, the first squeezing element 310 may be but is not limited to an arc-shaped liquid-stopping wall or other structures, the protrusion 322 may be a self-rotatable squeezing wheel, or a fixed arc or disc shape, or may have other shapes and structures of the arc-shaped second squeezing portion 321.

The arc-shaped second squeezing portion 321 can cooperate with the arc-shaped surface of the first squeezing portion 311 to squeeze the pipeline 210 against the arc-shaped surface of the first squeezing portion 311, which arc-shaped surface is concave.

In one embodiment, a second driving component is further included, which is connected with the roller component to drive the roller component to rotate, so as to enable the second squeezing portion 321 to move along an extension direction of the pipeline 210 (i.e., along a direction of the curved surface), when squeezing the pipeline 210, so as to push the fluid to flow. For example, in a state shown on the right side of FIG. 1, the roller component is driven to roll, thereby pushing the fluid inside the pipeline 210 to flow. A number of the protrusion(s) 322 on the roller component may be one or more. When multiple protrusions 322 exist, they may be arranged at intervals on one seat 323. The seat 323 can be a rotating wheel or other structures.

The second driving component and the first driving component 390 can be driven by a same driving element 131, or each can have its own driving element. For example, the second driving component and the first driving component 390 are driven by separate motors, respectively.

In some embodiments, when the first driving component 390 drives the second squeezing element 320 to move, it can also drive the second driving component to move together with the second squeezing element 320, so as to facilitate the second driving component to drive the second squeezing element 320 to rotate.

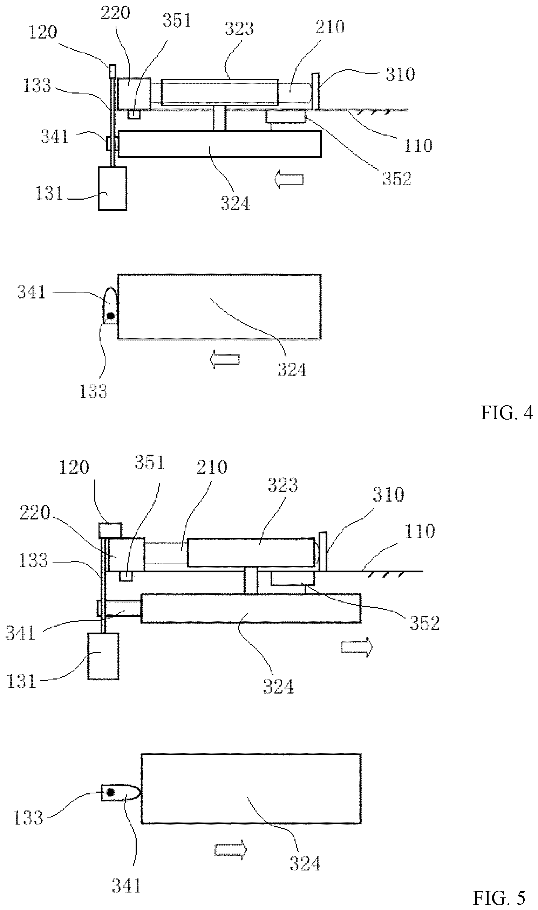

More specifically, in one embodiment, referring to FIGS. 4 and 5, the second squeezing element 320 includes a holder 324, and the roller component is assembled at the holder 324, and may be assembled at the holder 324 via a seat 323. The first driving component 390 is connected with the second squeezing element 320. The driving component includes a motor 131, which is connected with the holder 324, drives the holder 324, and brings the roller component to move toward a side where the first squeezing element 310 is located.

Furthermore, in this embodiment, an output shaft of the motor 131 is connected with the transmission cam 341, and the transmission cam 341 is connected with the holder 324. When the transmission cam 341 rotates, it can bring the holder 324 to move closer to and away from the first squeezing element 310. The transmission cam 341 can form a reciprocating linkage structure with the holder 324, that is, the second squeezing element 320 can be driven to move close to and away from the first squeezing element 310 through the rotation of the transmission cam 341, thereby automatically locking the pipeline to stop the liquid and releasing the pipeline 210.

On the other hand, the present embodiment also provides a locking apparatus for a pipeline component of a medical surgical pump, which locking apparatus is configured to achieve a locking effect on the pipeline component of the medical surgical pump, so as to prevent the pipeline component 200 from affecting a surgery by disturbance, and prevent the pipeline component from falling off. The pipeline component may include one or more pipelines 210 for a fluid to flow, and these pipelines 210 can be directly locked at the locking apparatus for a pipeline component. Alternatively, the pipeline component may also include a fixing block 220, which stores the pipelines 210 together, and then the fixing block 220 is locked by the locking apparatus for a pipeline component, thereby indirectly locking the pipeline 210.

Please refer to FIG.S 6-9. In one embodiment, the locking apparatus for a pipeline component 100 includes a base 110, a locking element 120 and a third driving component (not shown).

The base 110 has a placement region, which is configured to place the pipeline component 200. The base 110 may be a part of the housing of the medical surgical pump, or may be other structure(s) in the medical surgical pump that is(are) separate from the housing, as long as a placement region for placing the pipeline component 200 can be provided.

The third driving component is connected with the locking element 120 to drive the locking element 120 to move between a locking position and an unlocking position. The third driving component may use a driving element 131 in various forms, such as a motor, a cylinder, a hydraulic cylinder, an electromagnet, etc., to output a force and a movement, which is capable of changing a position of the locking element 120.

The third driving component and the first driving component 390 can be driven by the same driving element 131 to simplify the structure, so as to make the surgical pump more compact and smaller in size. Moreover, through the same driving element 131, the linkage between the locking apparatus for a pipeline component 100 and the pipeline fixing apparatus 300 can also be realized. As shown in FIG. 5, when the third driving element drives the locking element 120 to move to the locking position, the first driving element 390 also simultaneously drives the first squeezing element 310 and/or the second squeezing element 320 to clamp the pipeline 210. As shown in FIG. 4, when the third driving component drives the locking element 120 to move to the unlocking position, the first driving component 390 also simultaneously drives the first squeezing element 310 and/or the second squeezing element 320 to release the pipeline 210.

The connection between the third driving component and the locking element 120 is transmission connection, which is capable of implementing a transmission of force and movement. This connection includes direct or indirect contact connection, as well as contactless connection, as long as it can achieve the transmission of force and movement.

A locking position and an unlocking position exist on a moving track of the locking element 120, and the locking position and the unlocking position are usually a section of the moving track, and rather than simply refer to one position. When the locking element 120 is at the locking position, the locking element 120 fixes the pipeline component 200 inside the placement region. When the locking element 120 is at the unlocking position, the locking element 120 releases the pipeline component 200. That is, a position, where as long as the locking element 120 is capable of locking the pipeline component 200 inside the placement region, can be regarded as the locking position of the locking element 120. On the contrary, a position, where as long as the locking element 120 is capable of unlocking the pipeline component 200, can be regarded as the unlocking position of the locking element 120. For example, in the left-side structures of FIG. 4 and FIG. 6 and the state shown in FIG. 8, the locking element 120 releases the pipeline component 200. At this time, the locking element 120 is at the unlocking position, and the pipeline component 200 can be disassembled. In the right-side structures of FIG. 5 and FIG. 6 and the state shown in FIG. 9, the locking element 120 locks the pipeline component 200. At this time, the locking element 120 is at the locking position, and the locking element 120 can lock the pipeline component 200 by, but is not limited to, pressing, clamping, etc.

Without conflicting with other structures, the locking element 120 can move between the locking position and the unlocking position in any manner. For example, in one embodiment, the movement of the locking element 120 between the locking position and the unlocking position includes but is not limited to translation, rotation, or a combination thereof. The translation may include translation along a straight line, translation along a curve, translation along a circular track, or translation along various other tracks.

The locking element 120 is configured to lock and unlock the pipeline component 200. The locking element 120 may have a shape that can match the pipeline component 200, that is, it can lock the pipeline component 200 alone or in cooperation with other components (such as the base 110 ). The locking element 120 may be an integrally formed structure or a structure formed by combining two or more sub-components. For example, in FIGS. 4-9, the locking element 120 is a handle-shaped structure. Of course, the locking element 120 is not limited to the shape and structure shown in the figure.

The control unit 380 can control a state of the third driving component, and further control a movement position of the locking element 120. The control process of the control unit 380 on the third driving component is a conventional method and is not elaborated here.

Furthermore, in order to determine whether a pipeline component 200 exists inside the placement region, in one embodiment, a second in-position detection unit for detecting the pipeline component 200 inside the placement region, is further provided. The second in-position detection unit is connected with the control unit 380, and the control unit 380 is connected with the third driving component. When the second in-position detection unit detects that the pipeline component 200 exists inside the placement region, it feeds this back to the control unit 380, and then the control unit 380 controls the third driving component to move the locking element 120 to the locking position, so as to lock the pipeline component 200. The second in-position detection unit can detect the pipeline 210 in various ways and principles, such as but not limited to an optical coupler, a potentiometer, a magnetic angle sensor and the likes. The second in-position detection unit may be arranged inside the placement region or in other places outside the placement region, mainly for the purpose of detecting whether the pipeline component 200 exists inside the placement region.

On the other hand, the movement of the locking element 120 may also be triggered by the user. For example, in one embodiment, a trigger unit 370 for inputting locking and/or unlocking instruction(s) is also included (the trigger unit 370 of the pipeline fixing apparatus 300 may be shared or a separate trigger unit 370 may be provided), and the trigger unit 370 is connected with the control unit 380. When the user triggers the trigger unit 370, the control unit 380 controls the third driving component to move the locking element 120 to the locking position, so as to lock the pipeline component 200, and/or move the locking element 120 to the unlocking position, so as to unlock the pipeline component 200.

Furthermore, the third driving component includes a driving element 131 and a transmission element 133, wherein the driving element 131 is connected with the transmission element 133, the locking element 120 is connected with the transmission element 133, and the transmission element 133 transmits the movement, which is outputted by the driving element 131, to the locking element 120, so as to drive the locking element 120 to move between the locking position and the unlocking position. The driving element 131 is not limited to a motor, a cylinder, a hydraulic cylinder, an electromagnet, etc. The transmission element 133 can adopt a common transmission structure, such as shaft-transmission, synchronous belt-transmission mechanism, gear-transmission mechanism and other transmission mechanisms and their combinations.

As shown in FIG. 7, in one embodiment, the driving element 131 is a motor, the transmission element 133 includes a rotating shaft, which is connected with an output end of the motor, the locking element 120 is assembled at the rotating shaft, and the motor drives the locking element 120 to rotate, so as to switch between the locking position and the unlocking position. In addition, in FIG. 7, the transmission element 133 further includes a reduction gear box 132, one end of which is connected with the output end of the motor, and the other end of which is connected with the rotating shaft, thereby playing a role of speed reduction. The entire third driving component can be assembled at the housing of the medical surgical pump or other locations through the holder 324.

Further, please refer to FIG. 7. In one embodiment, the base 110 is a side housing of an outer housing of the medical surgical pump. The housing can be a front housing of the outer housing (a side of the outer housing facing the user, when the medical surgical pump is in normal use), or a rear housing of the outer housing (a side opposite to the front housing), a side housing of the outer housing (a housing between the front housing and the rear housing ), a top housing or a bottom housing of the outer housing. The driving element 131 is arranged inside the medical surgical pump housing, so that the driving element 131 can be accommodated inside the housing to avoid being exposed, which not only protects the driving element 131 but also ensures a neat appearance. The locking element 120 is arranged outside the base 110 to lock the pipeline component 200 from the outside of the base 110, making it convenient for user to disassemble and assemble the pipeline component 200.

In one embodiment, please refer to FIG. 7, the base 110 is a front housing of the medical surgical pump, and the placement region is located at an outer wall of the front housing. Because the front housing faces the user when the medical surgical pump is in normal use, the placement region is provided at the front housing to further facilitate the user to assemble and disassemble the pipeline component 200.

As shown in FIG. 7, in one embodiment, one end of the rotating shaft is located inside the outer housing, and the other end of the rotating shaft passes through the outer housing (such as the base 110) and extends out of the outer housing, and the locking element 120 is arranged at a portion of the rotating shaft, which portion extends out of the outer housing. This design can accommodate most of the third driving component within the outer housing, with only the locking element 120 or a portion of the rotating shaft being exposed. Of course, in some embodiments, a portion of the locking element 120 can also pass through the base 110 and extend into the outer housing. At this time, the shaft itself can be retained in the outer housing as a whole, so as to enable a connection portion between the locking element 120 and the shaft to be accommodated inside the housing, so as to prevent accidental touching which affects a connection effect between the locking element 120 and the shaft, and meanwhile to enable the entire structure to be neater and more beautiful.

Furthermore, as shown above, the locking element 120 can lock the pipeline component 200 by means of, but not limited to, pressing, clamping, and the likes. Please continue to refer to FIG. 7. In one embodiment, at the locking position, the locking element 120 and an outer side of the base 110 form a position-limiting space for clamping the pipeline component 200. The position-limiting space is configured to accommodate the pipeline component 200, such as the fixing block 220. Furthermore, a thickness defined by the position-limiting space (a distance between the locking element 120 and the base 110) is slightly smaller than a thickness of the fixing block 220, so the fixing block 220 can be clamped tight to the base 110.

Please refer to FIG. 7. In one embodiment, the locking element 120 has a guide portion 121 which is thin at front and thick at rear. The guide portion 121 has an inclined guide surface (a surface of the guide portion 121 facing outward). The front refers to an end of the locking element 120 away from the transmission element 133 (such as a rotating shaft), and the rear refers to an end of the locking element 120 connected with the transmission element 133 (such as a rotating shaft). The thickness refers to a dimension along an axial direction of the rotating shaft. When the fixing block 220 has a side opening 222, a thinner portion at the front end can more easily extend into the fixing block 220 from the side opening 222 during the rotation of the locking element 120.

On the other hand, this embodiment further provides a medical surgical pump, which includes a pipeline fixing apparatus 300 and/or a locking apparatus for a pipeline component 100 as shown in any of the above embodiments. The pipeline fixing apparatus 300 can stop the liquid and lock the pipeline 210. The locking apparatus for a pipeline component 100 can lock the pipeline component 200 tight to the base 11.

Please refer to FIGS. 6-9, the pipeline component 200 includes a pipeline 210 and a fixing block 220, and the pipeline 210 is assembled at the fixing block 220, and such assembly can be detachable connection or non-detachable connection. The fixing block 220 has a shape which matches the placement region, that is, the fixing block 220 can be stably placed inside the placement region. For example, the fixing block 220 has a contact surface or structure which corresponds to the shape of the placement region. At the locking position, the locking element 120 fixes the fixing block 220 inside the placement region; while at the unlocking position, the locking element 120 releases the fixing block 220.

In addition, the pipeline component 200 may not include the fixing block 220, and the locking apparatus for a pipeline component 100 directly locks the pipeline 210 in the pipeline component 200.

More specifically, please refer to FIGS. 6-9. In one embodiment, at the locking position, the locking element 120 abuts the fixing block 220 against the base 110, thereby locking the fixing block 220 by an abutting manner. At the unlocking position, the locking element 120 is disengaged from the fixing block 220, thereby releasing the fixing block 220.

In one embodiment, the fixing block 220 may be provided with a bottom plate 221 and a side opening 222. Please refer to FIGS. 6-9. When the locking element 120 moves towards the locking position, it extends from the side opening 222 of the fixing block 220 to the bottom plate 221 and abuts the bottom plate 221 against the base 110 for fixation. This method allows the locking element 120 to be located inside the fixing block 220. Compared with locking the fixing block 220 from the outermost side of the fixing block 220, this method can decrease a thickness of the fixing block 220 and the locking element 120 as a whole (protrusion 322), which protrudes outward from the base 110.

In one embodiment, the bottom plate 221 of the fixing block 220 has an avoidance opening 223, and the locking element 120 is located at the avoidance opening 223 to avoid mutual interference with the fixing block 220. The locking element 120 is accommodated inside the fixing block 220 through the avoidance opening 223, which further decreases a region occupied by the fixing block 220 and the locking element 120 on the base 110.

The pipeline component 200 can be manufactured and sold together with a medical surgical pump. Of course, in other embodiments, the medical surgical pump may not include the pipeline component 200, and the pipeline component 200 is a separate consumable, such as the relationship between a printer and printing papers.

In addition, an embodiment of this disclosure provides a control method which is applied to a pipeline of a medical surgical pump, including following steps.
controlling at least one of a first squeezing element 310 and a second squeezing element 320 to move toward the other, so as to clamp the pipeline, when a first trigger signal is received; specifically, the control unit 380 receives a first trigger signal, and controls, according to the first trigger signal, at least one of the first squeezing element 310 and the second squeezing element 320 to move toward the other, so as to clamp the pipeline; and/or
controlling at least one of a first squeezing element and a second squeezing element to move away from the other, so as to release the pipeline, when a second trigger signal is received; specifically, the control unit 380 receives the second trigger signal, and controls, according to the second trigger signal, at least one of the first squeezing element 310 and the second squeezing element 320 to move away from the other, so as to release the pipeline.

The above steps can be implemented by structure(s) shown in the above embodiments. For example, the first trigger signal and the second trigger signal can be implemented by method(s) shown in the above embodiments. The control unit 380 controls the first squeezing element 310 and the second squeezing element 320 to clamp and release the pipeline in a manner that can also be implemented in manner(s) shown in the above embodiments.

Of course, the above steps may also be implemented through other feasible structures and are not limited to the structures disclosed in the above embodiments.

The first trigger signal and the second trigger signal can be triggered by a user input and/or an automatic detection of the medical surgical pump.

For example, in one embodiment, the control method further includes:
determining that the first trigger signal is received, when it is detected that the pipeline is being assembled into a pipeline accommodating region of the medical surgical pump; and/or
determining that the second trigger signal is received, when it is detected that the pipeline is being disassembled out of a pipeline accommodating region of the medical surgical pump.

For example, the first trigger signal includes a trigger signal for assembling a pipeline. Whether the pipeline is being assembled inside the pipeline accommodating region of the medical surgical pump, is detected. If yes, the trigger signal for assembling a pipeline is transmitted to the control unit 380; and the control unit 380 receives the trigger signal for assembling a pipeline, controls at least one of the first squeezing element 310 and the second squeezing element 320 to move toward the other, so as to clamp the pipeline.

The second trigger signal includes a trigger signal for disassembling a pipeline. Whether the pipeline is being disassembled out of the pipeline accommodating region, is detected. If yes, the trigger signal for disassembling a pipeline is transmitted to the control unit 380; and the control unit 380 receives the trigger signal for disassembling a pipeline, controls at least one of the first squeezing element 310 and the second squeezing element 320 to move away from the other, so as to release the pipeline.

For another example, in one embodiment, the control method further includes:
determining that the first trigger signal is received, when an instruction for assembling a pipeline is received; and/or
determining that the second trigger signal is received, when an instruction for disassembling a pipeline is received.

For example, the first trigger signal includes a trigger signal for assembling a pipeline. When an instruction for assembling a pipeline is received, the trigger signal for assembling a pipeline is transmitted to the control unit 380; and the control unit 380 receives the trigger signal for assembling a pipeline, controls at least one of the first squeezing element 310 and the second squeezing element 320 to move toward the other, so as to clamp the pipeline.

The second trigger signal includes a trigger signal for disassembling a pipeline. When an instruction for disassembling a pipeline is received, the trigger signal for disassembling a pipeline is transmitted to the control unit 380; and the control unit 380 receives the trigger signal for disassembling a pipeline, controls at least one of the first squeezing element 310 and the second squeezing element 320 to move away from the other, so as to release the pipeline.

On the other hand, an embodiment of this disclosure provides a computer-readable storage medium, including a program(s), which is/are capable of being executed by a processor, so as to implement any of the control methods described above.

The above specific examples, which illustrate this disclosure, are only configured to help understand this disclosure rather than intended to limit this disclosure. For those skilled in the art of the technical field to which this disclosure belongs, several simple deductions, deformations or substitutions can be made according to the idea of this disclosure.

## Claims

1. A medical surgical pump, **characterized in that**, comprising a housing, a control unit, a first driving component, a first squeezing element, and a second squeezing element;
the housing comprises a panel, wherein the first squeezing element and/or the second squeezing element are/is movably arranged on the panel; the control unit is connected with the first driving component, and the first driving component is further connected with the first squeezing element and/or the second squeezing element; wherein a gap, which is configured to accommodate a pipeline, exists between the first squeezing element and the second squeezing element;
the control unit is configured to control the first driving component to drive at least one of the first squeezing element and the second squeezing element to move toward the other, so as to decrease the gap for fixing the pipeline, when receiving a first trigger signal; and/or the control unit is configured to control the first driving component to drive at least one of the first squeezing element and the second squeezing element to move away from the other, so as to increase the gap for releasing the pipeline, when receiving a second trigger signal.

2. The medical surgical pump according to claim 1, **characterized in that**, further comprising at least one first in-position detection unit, and the first trigger signal comprises a trigger signal for assembling a pipeline;
the first in-position detection unit is configured to transmit the trigger signal for assembling a pipeline to the control unit, when detecting that the pipeline is being assembled;
the control unit is further configured to control the first driving component to drive at least one of the first squeezing element and the second squeezing element to move toward the other, so as to decrease the gap, when receiving the trigger signal for assembling a pipeline.

3. The medical surgical pump according to claim 1, **characterized in that**, further comprising at least one first in-position detection unit, and the second trigger signal comprises a trigger signal for disassembling a pipeline;
the first in-position detection unit is configured to transmit the trigger signal for disassembling a pipeline to the control unit, when detecting that the pipeline is being disassembled;
the control unit is further configured to control the first driving component to drive at least one of the first squeezing element and the second squeezing element to move away from the other, so as to increase the gap, when receiving the trigger signal for disassembling a pipeline.

4. The medical surgical pump according to claim 2 or claim 3, **characterized in that**, the first in-position detection unit is arranged inside a pipeline accommodating region of the medical surgical pump.

5. The medical surgical pump according to claim 1, **characterized in that**, further comprising a unit for inputting a user instruction, wherein the first trigger signal comprises a trigger signal for assembling a pipeline;
the unit for inputting a user instruction is configured to transmit the trigger signal for assembling a pipeline to the control unit, when receiving, from a user, an instruction for assembling a pipeline;
the control unit is further configured to control the first driving component to drive at least one of the first squeezing element and the second squeezing element to move toward the other, so as to decrease the gap, when receiving the trigger signal for assembling a pipeline.

6. The medical surgical pump according to claim 1, **characterized in that**, further comprising a unit for inputting a user instruction, wherein the second trigger signal comprises a trigger signal for disassembling a pipeline;
the unit for inputting a user instruction is configured to transmit the trigger signal for disassembling a pipeline to the control unit, when receiving, from a user, an instruction for disassembling a pipeline;
the control unit is further configured to control the first driving component to drive at least one of the first squeezing element and the second squeezing element to move away from the other, so as to increase the gap, when receiving the trigger signal for disassembling a pipeline.

7. The medical surgical pump according to claim 5 or claim 6, **characterized in that**, the unit for inputting a user instruction comprises at least one of a physical trigger element, a touch-control element, a sound acquisition unit, and a gesture acquisition unit.

8. The medical surgical pump according to claim 1, **characterized in that**, further comprising a trigger unit, wherein the first trigger signal comprises a trigger signal for assembling a pipeline, and the second trigger signal comprises a trigger signal for disassembling a pipeline;
the trigger unit is configured to transmit the trigger signal for assembling a pipeline to the control unit, when detecting that the pipeline is being assembled or receiving from a user an instruction for assembling a pipeline; the control unit is further configured to control the first driving component to drive at least one of the first squeezing element and the second squeezing element to move toward the other, so as to decrease the gap, when receiving the trigger signal for assembling a pipeline; and/or
the trigger unit is configured to transmit the trigger signal for disassembling a pipeline to the control unit, when detecting that the pipeline is being disassembled or receiving from a user an instruction for disassembling a pipeline; the control unit is further configured to control the first driving component to drive at least one of the first squeezing element and the second squeezing element to move away from the other, so as to increase the gap, when receiving the trigger signal for disassembling a pipeline.

9. The medical surgical pump according to claim 1, **characterized in that**, further comprising a position detection unit, which is arranged on moving track(s) of the first squeezing element and/or the second squeezing element, wherein the position detection unit is connected with the control unit;
the position detection unit is configured to transmit an in-position signal to the control unit, when detecting that the first squeezing element and/or the second squeezing element move/moves into position; and
the control unit is further configured to control the first driving component to stop driving the first squeezing element and/or the second squeezing element to move, when receiving the in-position signal.

10. The medical surgical pump according to any one of claims 1-9, **characterized in that**, at least a section of a relative movement between the first squeezing element and the second squeezing element is a translational movement.

11. The medical surgical pump according to any one of claims 1-10, **characterized in that**, the first squeezing element comprises an arc-shaped surface, and the second squeezing element comprises a roller component;
the roller component comprises a rotating wheel and a plurality of squeezing wheels, wherein the squeezing wheels are arranged around the rotating wheel.

12. The medical surgical pump according to claim 11, **characterized in that**, further comprising a second driving component, which is connected with the roller component;
the second driving component is configured to drive the rotating wheel to rotate, so as to enable the squeezing wheels, when abutting against the pipeline, to move along the arc-shaped surface, thereby pushing a fluid inside the pipeline to flow.

13. The medical surgical pump according to claim 12, **characterized in that**, the second squeezing element further comprises a holder, wherein the roller component is assembled at the holder, the first driving component comprises a motor and a transmission cam, an output shaft of the motor is connected with the transmission cam, the transmission cam is further connected with the holder, wherein a rotation of the transmission cam is capable of driving the holder to move toward and/or away from the first squeezing element.

14. The medical surgical pump according to any one of claims 1-13, **characterized in that**, further comprising a locking element and a third driving component, wherein the housing is provided with a base, and the base is provided with a placement region, which region is configured to place a pipeline component; wherein the pipeline component comprises the pipeline and a fixing block, wherein the pipeline is assembled at the fixing block, and the locking element is configured to lock and unlock the fixing block of the pipeline component; wherein the third driving component is connected with the locking element, so as to drive the locking element to move between a locking position and an unlocking position;
when the locking element is at the locking position, the locking element and the placement region form a position-limiting space, which space is capable of accommodating the pipeline component, and the locking element is capable of locking the fixing block inside the position-limiting space; when the locking element is at the unlocking position, the locking element unlocks the fixing block.

15. The medical surgical pump according to claim 14, **characterized in that**, when the locking element is switched to the locking position, the first trigger signal is transmitted to the control unit; and/or when the locking element is switched to the unlocking position, the second trigger signal is transmitted to the control unit.

16. The medical surgical pump according to claim 14, **characterized in that**, further comprising a second in-position detection unit, which is connected with the control unit;
the second in-position detection unit is configured to trigger the control unit to control the third driving component, so as to drive the locking element to move to the locking position, when detecting that the pipeline component is arranged inside the placement region.

17. A control method which is applied to a pipeline of a medical surgical pump, **characterized in that**, comprising:
controlling at least one of a first squeezing element and a second squeezing element to move toward the other, so as to clamp the pipeline, when a first trigger signal is received; and/or
controlling at least one of a first squeezing element and a second squeezing element to move away from the other, so as to release the pipeline, when a second trigger signal is received.

18. The control method according to claim 17, **characterized in that**, further comprising
determining that the first trigger signal is received, when it is detected that the pipeline is being assembled into a pipeline accommodating region of the medical surgical pump; and/or
determining that the second trigger signal is received, when it is detected that the pipeline is being disassembled out of a pipeline accommodating region of the medical surgical pump.

19. The control method according to claim 17, **characterized in that**, further comprising
determining that the first trigger signal is received, when an instruction for assembling a pipeline is received; and/or
determining that the second trigger signal is received, when an instruction for disassembling a pipeline is received.

20. A computer-readable storage medium, **characterized in that**, comprising program(s), which is/are capable of being executed by a processor, so as to implement the control method according to any one of claims 17-19.
